# EUROPEAN PATENT APPLICATION

(11) **EP 4 501 402 A1**
(43) Date of publication of application: **05.02.2025**
(21) Application number: 23779091.0
(22) Date of filing: 24.02.2023
(51) Int. Cl.: A61P 1/02, A61Q 11/00, A61K 8/67, A61K 31/375

(54) **COMPOSITION CONTAINING DL-ALPHA-TOCOPHEROL 2-L-ASCORBIC ACID PHOSPHORIC ACID DIESTER ALKALI METAL SALT**

(30) Priority: 30.03.2022 JP 2022055850; 30.03.2022 JP 2022055854
(71) Applicant: SUNSTAR INC., Takatsuki-shi, Osaka 569-1195 (JP)
(72) Inventor: MORIKAWA, Takuma, Takatsuki-shi, Osaka 569-1195 (JP); HANADA, Sakae, Takatsuki-shi, Osaka 569-1195 (JP); URAKAWA, Rika, Takatsuki-shi, Osaka 569-1195 (JP); ARITA, Takuya, Takatsuki-shi, Osaka 569-1195 (JP)
(74) Representative: Müller-Boré & Partner Patentanwälte PartG mbB
(86) International application number: PCT/JP2023/006641
(87) International publication number: WO 2023/189061

(57) **Abstract**

Provided is an oral composition comprising an L-ascorbic acid dl-α-tocopherol phosphoric acid diester alkali metal salt. The composition according to the present disclosure is capable of suppressing disruption of the oral mucosal epithelial barrier function and/or inhibiting the activity of matrix metalloproteinases (MMPs). Thus, the composition according to the present disclosure is capable of preventing the onset of periodontal disease and/or suppressing the progression of periodontal disease.

## Description

### Technical Field

The present disclosure relates to a composition comprising an L-ascorbic acid dl-α-tocopherol phosphoric acid diester alkali metal salt, and the like.

### Background Art

L-Ascorbic acid dl-α-tocopherol phosphoric acid diester (also referred to as "EPC" in the present specification) is a compound in which ascorbic acid (vitamin C) and tocopherol (vitamin E) are ester-bonded via phosphoric acid. L-Ascorbic acid dl-α-tocopherol phosphoric acid diester has anti-oxidative action and moisturizing action, and is used in cosmetics such as hair restorers (Patent Literature (PTL) 1).

There are no reports yet on the effect of EPC in the oral region.

### Citation List

### Patent Literature

PTL 1: WO2020/196852

### Non-patent Literature

NPL 1: Periodontol 2000. 2015 Oct; 69 (1): 7-17. Molecular aspects of the pathogenesis of periodontitis
NPL 2: J Periodontal Res. 2016 Dec; 51 (6): 748-757. Influence of retinoic acid on human gingival epithelial barriers
NPL 3: Int J Dent. 2012; 2012: 821383. Published online 2012 Jul 26. Host-Bacteria Crosstalk at the Dentogingival Junction
NPL 4: Arch Oral Biol. 2016 Jun; 66: 30-7. The traditional Japanese medicine hangeshashinto alleviates oral ulcer-induced pain in a rat model.
NPL 5: Aust Dent J. 2009 Dec; 54 (4): 347-54. Localization of matrix metalloproteinases (MMPs-2, 8, 9, and 20) in normal and carious dentine.

### Summary of Invention

### Technical Problem

An object of the present disclosure is to provide a composition capable of preventing the onset of periodontal disease and/or suppressing the progression of periodontal disease.

### Solution to Problem

The present inventors found that an L-ascorbic acid dl-α-tocopherol phosphoric acid diester alkali metal salt suppresses disruption of the oral mucosal epithelial barrier function and inhibits the activity of matrix metalloproteinases (MMPs). The inventors then made further improvements.

The present disclosure encompasses, for example, the subject matter described in the following Items.

### Item 1.

An oral composition comprising an L-ascorbic acid dl-α-tocopherol phosphoric acid diester alkali metal salt, with the proviso that an oral composition comprising at least one member selected from the group consisting of polyoxyethylene alkyl phenyl ethers, polyoxyethylene alkyl ethers, and polyoxyethylene fatty acid esters is excluded.

### Item 2.

An anti-periodontal disease composition comprising an L-ascorbic acid dl-α-tocopherol phosphoric acid diester alkali metal salt.

### Item 3.

The composition according to Item 1 or 2, comprising 0.001 to 1 mass% of the L-ascorbic acid dl-α-tocopherol phosphoric acid diester alkali metal salt.

### Item 4.

The composition according to any one of Items 1 to 3, wherein the L-ascorbic acid dl-α-tocopherol phosphoric acid diester alkali metal salt is L-ascorbic acid dl-α-tocopherol phosphoric acid diester potassium salt.

### Item 5.

The composition according to any one of Items 1 to 4, for use in anti-oral mucositis or anti-caries.

### Item 6.

The composition according to any one of Items 1 to 5, for use in enhancing an oral mucosal epithelial barrier function.

### Item 7.

The composition according to any one of Items 1 to 6, for use in inhibiting matrix metalloproteinase activity in the oral cavity.

### Item 8.

Use of an L-ascorbic acid dl-α-tocopherol phosphoric acid diester alkali metal salt for the production of an anti-periodontal disease composition, a composition for anti-oral mucositis, a composition for anti-caries, a composition for enhancing an oral mucosal epithelial barrier function, or a composition for inhibiting matrix metalloproteinase activity in the oral cavity.

### Item 9.

The use according to Item 8, wherein the L-ascorbic acid dl-α-tocopherol phosphoric acid diester alkali metal salt is L-ascorbic acid dl-α-tocopherol phosphoric acid diester potassium salt.

### Item 10.

The use according to Item 8 or 9, wherein the content of the L-ascorbic acid dl-α-tocopherol phosphoric acid diester alkali metal salt in the composition is 0.001 to 1 mass%.

### Advantageous Effects of Invention

The composition according to the present disclosure is capable of suppressing disruption of the oral mucosal epithelial barrier function and/or inhibiting MMP activity. Thus, the composition according to the present disclosure is capable of preventing the onset of periodontal disease and/or suppressing the progression of periodontal disease.

### Brief Description of Drawings

Fig. 1 shows the permeability (%) when EPC-K (L-ascorbic acid dl-α-tocopherol phosphoric acid diester potassium salt) was contained.
Fig. 2 shows the permeability (%) when EPC-K was contained.
Fig. 3 shows the permeability (%) when EPC-K was contained, when APM (L-ascorbic acid phosphate magnesium salt) was contained, when VC (ascorbic acid) was contained, when VEA (tocopherol acetate) was contained, and when VEN (tocopherol nicotinate) was contained.
Fig. 4 shows the results of measurement of MMP-8 activity.
Fig. 5 shows the results of measurement of MMP-1 activity.
Fig. 6 shows the cell viability (%) when sorbeth-60 tetraoleate was contained, when diethyl sebacate was contained, when polyoxyethylene (10) hydrogenated castor oil was contained, when polyoxyethylene (9) lauryl ether was contained, and when polyoxyethylene (20) stearyl ether was contained.
Fig. 7 shows the cell viability (%) when polyoxyethylene (9) lauryl ether was contained, when polyoxyethylene (20) cetyl ether was contained, when polyoxyethylene (20) stearyl ether was contained, when polyoxyethylene (10) octylphenyl ether was contained, and when polyoxyethylene glycol monolaurate was contained.

### Description of Embodiments

Embodiments encompassed by the present disclosure are described in more detail below.

The compositions encompassed by the present disclosure comprise an EPC alkali metal salt. In the present specification, these compositions may be referred to as "the composition according to the present disclosure."

L-Ascorbic acid dl-α-tocopherol phosphoric acid diester (EPC) is a compound in which ascorbic acid (vitamin C) and tocopherol (vitamin E) are ester-bonded via phosphoric acid, and is also referred to as "(ascorbyl/tocopheryl) phosphate." The chemical formula is shown below.

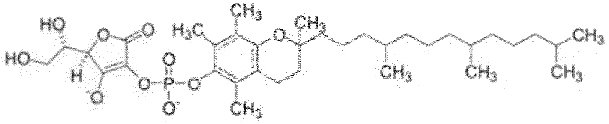

Examples of EPC alkali metal salts include EPC potassium salt, EPC sodium salt, EPC lithium salt, and the like. Of these, EPC potassium salt is preferable.

The EPC alkali metal salt may be a mono-salt or a di-salt.

The content of the EPC alkali metal salt in the composition according to the present disclosure may be, for example, about 0.001 to 1 mass%. The upper or lower limit of this range may be, for example, about 0.002, 0.005, 0.01, 0.02, 0.03, 0.04, 0.05, 0.06, 0.07, 0.08, 0.09, 0.1, 0.2, 0.3, 0.4, 0.5, 0.6, 0.7, 0.8, or 0.9 mass%. More specifically, the content of the EPC alkali metal salt in the composition according to the present disclosure may be, for example, about 0.002 to 0.5 mass%, or about 0.005 to 0.3 mass%.

Since the composition according to the present disclosure comprises an EPC alkali metal salt, the composition exhibits an effect of suppressing disruption of the oral mucosal epithelial barrier function. Thus, the composition according to the present disclosure can be suitably used for enhancing the oral mucosal epithelial barrier function. The oral mucosal epithelium is not particularly limited, and examples include epithelium (e.g., junctional epithelium and gingival epithelium, such as crevicular epithelium) constituting the masticatory mucosa (e.g., gingiva), epithelium constituting the lining mucosa (e.g., buccal mucosa), and epithelium constituting the specialized mucosa (e.g., lingual mucosa).

In addition, since the composition according to the present disclosure comprises an EPC alkali metal salt, the composition exhibits an effect of inhibiting MMP activity. Thus, the composition according to the present disclosure can be suitably used for inhibiting matrix metalloproteinase (MMP) activity in the oral cavity. The composition according to the present disclosure exhibits an MMP activity inhibitory effect and is therefore capable of inhibiting the activity of already produced neutrophil collagenase. Examples of matrix metalloproteinases (MMPs) in the oral cavity include MMP-1, MMP-2, MMP-3, MMP-8, MMP-9, MMP-13, and the like. Of these, MMP-1 and MMP-8 are preferable. MMP-1 (EC 3.4.24.7) is also referred to as "interstitial collagenase." MMP-8 (EC 3.4.24.34) is also referred to as "neutrophil collagenase."

It has been reported that the gingival epithelium forms a barrier against invading microorganisms and protects periodontal tissue from infection (NPL 1). Periodontal pathogens such as *Porphyromonas gingivalis* are known to achieve entry into periodontal tissue by secreting a bacterial protease and disrupting the gingival epithelium (NPL 2). It is known that as periodontal pathogens invade the tissue, immune cells such as neutrophils accumulate to eliminate them, and the production of MMPs increases. MMPs are proteolytic enzymes that degrade the extracellular matrix (e.g., collagen, such as Type I collagen, Type II collagen, and type III collagen), and disrupt periodontal tissue to cause progression of periodontal disease. Indeed, increased amounts of MMP-1, MMP-2, MMP-3, MMP-8, MMP-9, and MMP-13 have been found in gingival crevicular fluid (GCF) of patients with periodontal disease (NPL 3).

Since the composition according to the present disclosure comprises an EPC alkali metal salt, the composition exhibits an effect of suppressing disruption of the oral mucosal epithelial barrier function and/or an effect of inhibiting MMP activity and thus can be suitably used as an oral composition, especially for anti-periodontal disease. That is, the composition according to the present disclosure can prevent the progression of periodontal pathogens to periodontal tissue by enhancing the oral mucosal epithelial barrier function. The composition according to the present disclosure can also prevent disruption of periodontal tissue by inhibiting matrix metalloproteinase activity in the oral cavity. Thus, the composition according to the present disclosure is expected to be applicable to all conditions of periodontal disease, from early to advanced stages. In the present specification, the term "anti-periodontal disease" means prevention of the onset of periodontal disease and/or suppression of the progression of periodontal disease.

It has also been reported that when ulcers form in the oral mucosa due to, for example, bites, burns, or side effects of chemotherapy or radiotherapy, the epithelial barrier function is lost, causing oral bacterial infection (oral mucositis; NPL 4). Since the composition according to the present disclosure comprises an EPC alkali metal salt, the composition exhibits an effect of suppressing disruption of the oral mucosal epithelial barrier function and thus can be suitably used for anti-oral mucositis. In the present specification, the term "anti-oral mucositis" means prevention of the onset of oral mucositis and/or suppression of the progression of oral mucositis.

Dentin contains about 20% collagen, which is an organic substance, and the progression of dentin caries is accompanied by demineralization and collagen degradation. Regarding collagen degradation, MMPs in saliva are considered to penetrate dentin and degrade collagen (NPL 5). Since the composition according to the present disclosure comprises an EPC alkali metal salt, the composition exhibits an effect of inhibiting MMP activity and thus can be suitably used for anti-caries. In the present specification, the term "anti-caries" means prevention of the onset of caries and/or suppression of the progression of caries.

Examples of subjects to which the composition according to the present disclosure can be applied include mammals, including humans (e.g., dogs, cats, mice, rats, sheep, horses, bovine, and monkeys), and the like. Of these, humans are preferable.

Examples of humans to which the composition according to the present disclosure can be applied include patients with periodontal disease or humans suspected of having periodontal disease; patients with oral mucositis or humans suspected of having oral mucositis; patients with caries or humans suspected of having caries; humans with reduced oral mucosal epithelial barrier function; humans with enhanced matrix metalloproteinase activity in the oral cavity; and the like. The humans to which the composition according to the present disclosure can be applied are not limited to these, and may also include those who are healthy (e.g., humans who want to prevent the onset of periodontal disease, humans who want to suppress the progression of periodontal disease, humans who want to prevent the onset of oral mucositis, humans who want to suppress the progression of oral mucositis, humans who want to prevent the onset of caries, and humans who want to suppress the progression of caries) and the like.

The composition according to the present disclosure can be, for example, a solid composition or a liquid composition. The composition (in particular, oral composition) according to the present disclosure can be made into a form (dosage form), such as an ointment, a paste, a dermatological paste, a gel, a liquid, a spray, a mouthwash, a liquid dentifrice, a toothpaste, a gum, a tablet, or a drop in accordance with an ordinary method. Of these, an ointment, a paste, a gel, a liquid, a mouthwash, a liquid dentifrice, and a toothpaste are preferred.

The composition according to the present disclosure may further comprises, in addition to the EPC alkali metal salt, for example, one or two or more optional components that can be added to oral compositions in the field, such as surfactants, flavoring agents, sweeteners, wetting agents, binders, preservatives, colorants, pH adjusters, and medicinal ingredients other than EPC.

For example, at least one surfactant selected from the group consisting of nonionic surfactants, anionic surfactants, and ampholytic surfactants, may be added. Specific examples of nonionic surfactants include sugar fatty acid esters, such as sucrose fatty acid esters, maltose fatty acid esters, and lactose fatty acid esters; fatty acid alkanolamides; glycerin fatty acid esters; sorbitan fatty acid esters; fatty acid monoglyceride; diethyl sebacate; polyoxyethylene hydrogenated castor oil; fatty acid polyoxyethylene sorbitan; and the like. Examples of anionic surfactants include sulfuric acid ester salts, such as sodium lauryl sulfate and sodium polyoxyethylene lauryl ether sulfate; sulfosuccinates, such as sodium lauryl sulfosuccinate and sodium polyoxyethylene lauryl ether sulfosuccinate; acyl amino acid salts, such as sodium cocoyl sarcosine and sodium lauroyl methylalanine; sodium cocoyl methyl taurine; and the like. Examples of ampholytic surfactants include betaine acetate activators, such as betaine lauryl dimethylamino acetate and coconut oil fatty acid amide propyldimethylamino acetate betaine; imidazoline activators, such as sodium N-cocoyl-N-carboxymethyl-N-hydroxyethylethylenediamine; and the like. These surfactants can be added singly or in a combination of two or more. The amount of surfactant added is typically 0.1 to 5 mass% based on the total amount of the composition.

The composition according to the present disclosure may not contain, for example, at least one surfactant selected from the group consisting of polyoxyethylene alkyl phenyl ethers, polyoxyethylene alkyl ethers, and polyoxyethylene fatty acid esters. In particular, the composition according to the present disclosure may not contain polyoxyethylene alkyl phenyl ethers, polyoxyethylene alkyl ethers, or polyoxyethylene fatty acid esters.

Examples of polyoxyethylene alkyl phenyl ethers include polyoxyethylene alkyl phenyl ethers in which the average addition mole number of ethylene oxide is 1 to 40 and the number of carbon atoms in the alkyl group is 7 to 30, and the like. Examples of polyoxyethylene alkyl ethers include polyoxyethylene alkyl ethers in which the average addition mole number of ethylene oxide is 1 to 40 and the number of carbon atoms in the alkyl group is 1 to 18, and the like. Examples of polyoxyethylene fatty acid esters include polyoxyethylene fatty acid esters in which the average addition mole number of ethylene oxide is 1 to 40 and the number of carbon atoms in the alkyl group is 1 to 20, and the like.

Examples of flavoring agents that can be added include menthol, carvone, anethole, eugenol, methyl salicylate, limonene, ocimene, n-decyl alcohol, citronellol, α-terpineol, methyl acetate, citronellyl acetate, methyleugenol, cineol, linalool, ethyl linalool, thymol, spearmint oil, peppermint oil, lemon oil, orange oil, sage oil, rosemary oil, cinnamon oil, beefsteak plant oil, wintergreen oil, clove oil, eucalyptus oil, pimento oil, d-camphor, d-borneol, fennel oil, cinnamon oil, cinnamaldehyde, mint oil, vanillin, and the like. These flavoring agents can be used singly or in a combination of two or more, and the amount of flavoring agent added may be, for example, 0.001 to 1.5 mass% based on the total amount of the composition.

Examples of sweeteners include saccharin sodium, acesulfame potassium, stevioside, neohesperidin dihydrochalcone, perillartine, thaumatin, aspartylphenylalanine methyl ester, p-methoxycinnamic aldehyde, and the like. These sweeteners can be used singly or in a combination of two or more, and the amount of sweetener added may be, for example, 0.01 to 1 mass% based on the total amount of the composition.

Further, wetting agents such as sorbitol, ethylene glycol, propylene glycol, glycerol, 1,3-butylene glycol, polypropylene glycol, xylitol, maltitol, lactitol, and polyoxyethylene glycol can be added singly, or in a combination of two or more.

Examples of binders include cellulose derivatives, such as sodium carboxymethyl cellulose, carboxy methyl ethyl cellulose salts, hydroxyethyl cellulose, hydroxypropyl cellulose, hydroxypropyl methylcellulose, methyl cellulose, ethyl cellulose, crystalline cellulose, and crystalline cellulose-carmellose sodium; microbial polymers, such as xanthan gum; natural polymers or natural rubber, such as gum tragacanth, gum karaya, gum arabic, carrageenan, dextrin, agar, pectin, pullulan, gellan gum, locust bean gum, and sodium alginate; synthetic polymers, such as polyvinyl alcohol, polyvinyl pyrrolidone, carboxy vinyl polymers, polyvinyl methyl ether, and sodium polyacrylate; inorganic binders, such as thickening silica and Veegum; and cationic binders, such O-[2-hydroxy-3-(trimethylammonio)propyl]hydroxyethyl cellulose chloride. These binders can be used singly or in a combination of two or more.

Preservatives such as the following can be added: parabens, such as methylparaben, ethylparaben, propylparaben, and butylparaben; sodium benzoate; phenoxyethanol; and alkyldiaminoethylglycine hydrochloride. These preservatives can be used singly or in a combination of two or more.

Colorants such as the following can be added: legally permitted pigments, such as blue No. 1, yellow No. 4, red No. 202, and green No. 3; mineral-based pigments, such as ultramarine, enhanced ultramarine, and ferric hexacyanoferrate; and titanium oxide. These colorants can be used singly or in a combination of two or more.

pH Adjusters such as the following can be added: citric acid, phosphoric acid, malic acid, pyrophosphoric acid, lactic acid, tartaric acid, glycerophosphoric acid, acetic acid, nitric acid, chemically possible salts thereof, and sodium hydroxide. These pH adjusters can be added singly or in a combination of two or more such that the composition has a pH of 4 to 8, and preferably 5 to 7. The amount of pH adjuster may be, for example, 0.01 to 2 mass%.

The composition according to the present disclosure may further comprise the following medicinal ingredients other than EPC alkali metal salts, singly or in a combination of two or more: vitamin E, such as dl-α-tocopherol acetate, tocopherol succinate, or tocopherol nicotinate; ampholytic sterilizers, such as dodecyl diamino ethyl glycine; nonionic sterilizers, such as triclosan and isopropyl methylphenol; anionic sterilizer, such as sodium lauroyl sarcosine; cationic sterilizers, such as cetylpyridinium chloride, chlorhexidine hydrochloride, benzalkonium chloride, and benzethonium chloride; enzymes, such as dextranase, amylase, protease, mutanase, lysozyme, and lytic enzymes; alkali metal monofluorophosphates, such as sodium monofluorophosphate and potassium monofluorophosphate; fluorides, such as sodium fluoride and stannous fluoride; tranexamic acid; epsilon aminocaproic acid; aluminum chlorohydroxy allantoin; and dihydrocholesterol, glycyrrhetinic acid, glycyrrhizic acid, sodium copper chlorophyllin, glycerophosphate, chlorophyll, sodium chloride, caropeptide, allantoin, carbazochrome, potassium nitrate, and palatinit.

Bases such as the following can also be added: alcohols, silicon, apatite, white Vaseline, paraffin, liquid paraffin, microcrystalline wax, squalane, and Plastibase. These bases can be used singly or in a combination of two or more.

The composition according to the present disclosure can be prepared according to a known method for producing this type of composition. The composition according to the present disclosure can be prepared, for example, by appropriately mixing an EPC alkali metal salt and, if necessary, other components.

In the present specification, the terms "comprising" and "containing" also includes "consisting essentially of" and "consisting of." The present disclosure encompasses any combination of the elements described in the present specification.

The various characteristics (e.g., properties, numerical values, functions) described in each embodiment of the present disclosure can be combined in any way in specifying the subject matter encompassed in the present disclosure. Specifically, the present disclosure encompasses all subject matter formed by any possible combination of the combinable characteristics described in the present specification.

### Examples

The present disclosure is described in detail below with reference to experimental examples; however, the present disclosure is not limited to these examples. In the following, experiments were performed at atmospheric pressure and ordinary temperature, unless otherwise specified. Unless otherwise specified, "%" indicates "mass%." Further, unless otherwise specified, the amount of each component shown in the figures also indicates "mass%."

### 1. Epithelial Barrier Function Evaluation Test

### Cell Preparation

Gingival epithelial cell line Epi4 cells were cultured on Transwell to confluency. At this time, the Epi4 cells were cultured in a medium in which to Epilife (Thermo Fisher), Supplemental S7 (Thermo Fisher) was added in a 1/100 volume. In the present disclosure, the medium may be referred to as a "medium for cells." Transwell is a tool that allows for the evaluation of cell permeability, designed so that the inserts are hung in the middle of the wells.

### Bacterial Preparation

A *P. g* bacterium (*Porphyromonas gingivalis* W83) was cultured in a modified GAM medium (Nissui Pharmaceutical Co., Ltd.) and collected by centrifugation at 10000 rpm after culture. The concentration of the collected *P. g* bacterium was adjusted with a medium for cells so that OD₆₀₀ = 1.0.

### Material Preparation

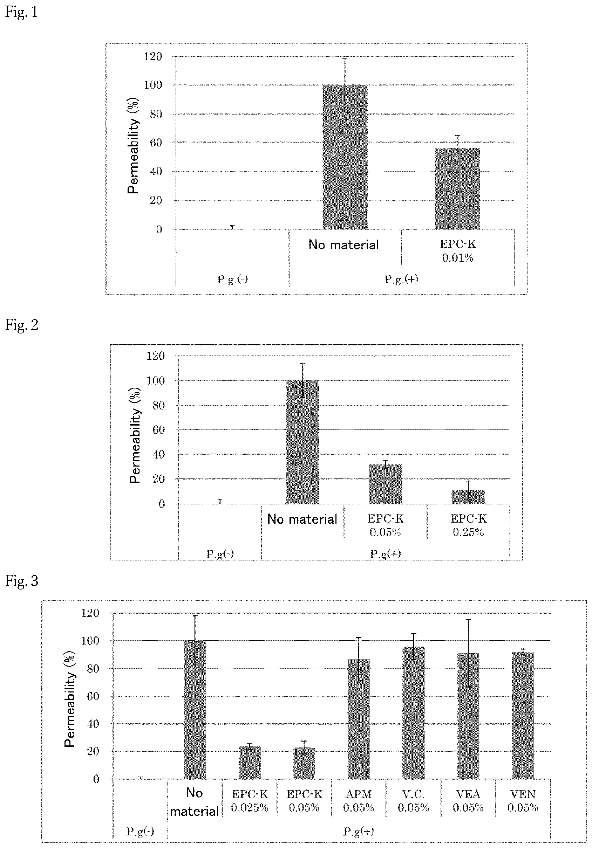

Each evaluation material was individually added to a 2.5% DMSO-containing medium for cells at the respective concentrations shown in Figs. 1 to 3 to prepare media containing each evaluation material. The evaluation materials used were EPC-K (L-ascorbic acid dl-α-tocopherol phosphoric acid diester potassium salt), APM (L-ascorbic acid phosphate magnesium salt), VC (ascorbic acid), VEA (tocopherol acetate), and VEN (tocopherol nicotinate).

### Barrier Function Evaluation

The medium for cells was removed from the Epi4 cells cultured on the Transwell, and each of the prepared evaluation material-containing media was individually added in an amount of 300 ul, followed by incubation at 37°C and a CO₂ concentration of 5 volume% for 1 hour. A sample in which a medium containing no evaluation material was added was used as a control.

Subsequently, 100 ul of the prepared *P. g* bacterium liquid was added, followed by incubation at 37°C and a CO₂ concentration of 5% for 2 hours. Regarding the control, those obtained by adding the *P. g* bacterium liquid and performing incubation (*P*. *g* (+)) and by adding only a medium containing no *P*. *g* bacterium and performing incubation (P. *g* (-)) were prepared.

After incubation, the medium was removed, washing was performed with PBS, and FITC (fluorescein isothiocyanate)-dextran (4 kDa) prepared at 1 mg/ml was added, followed by incubation at 37°C for 1 hour. FITC-dextran that passed through the Transwell was collected, and the fluorescence intensity (excitation light: 490 nm; emission light: 520 nm) was measured with a fluorescence plate reader (Gemini XPS). Based on the amount of FITC that permeated in the case in which the *P. g* bacterium liquid was added to the control (*P*. *g* (+); no material) taken as 100%, and the amount of FITC that permeated in the case in which only the medium containing no *P. g* bacterium was added to the control (*P*. *g* (-)) taken as 0%, the permeability in the case in which treatment was performed with each of the evaluation materials at the respective concentrations was calculated. Figs. 1 to 3 show the results. The concentration of each evaluation material in Figs. 1 to 3 is the concentration at the time of preparing each of the evaluation material-containing media, and the final concentration (concentration after addition of *P*. *g* bacterium) is 3/4 of this concentration value. The lower the permeability, the higher the epithelial barrier function.

As shown in Figs. 1 to 3, it was confirmed that EPC-K suppressed disruption of oral mucosal epithelial barrier caused by the *P*. *g* bacterium. Similar effects were not observed for ascorbic acid or its derivative APM, or tocopherol derivatives VEA or VEN.

### 2. MMP Activity Measurement Test

### Material Preparation

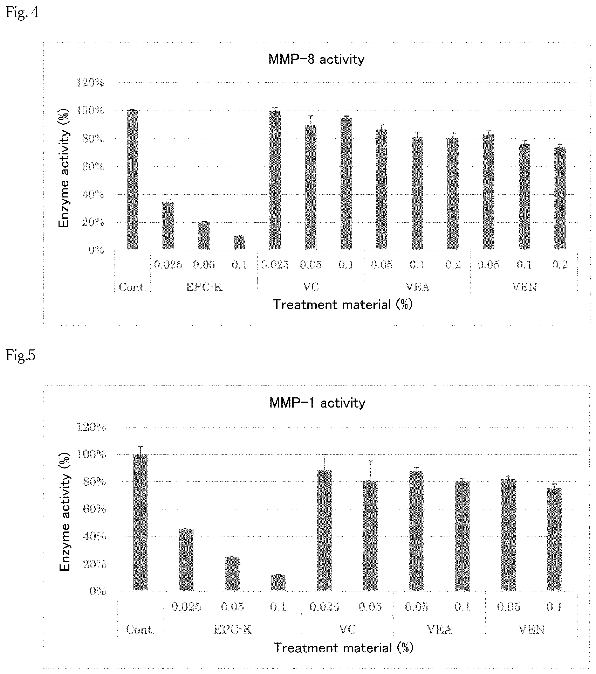

Evaluation material solutions were prepared by individually adding each evaluation material at the respective concentrations shown in Figs. 4 and 5 to a solvent in which 5%DMSO was added to the buffer included in each kit below. The evaluation materials used are EPC-K (L-ascorbic acid dl-α-tocopherol phosphoric acid diester potassium salt), VC (ascorbic acid), VEA (tocopherol acetate), and VEN (tocopherol nicotinate).

### Enzyme Activity Measurement

MMP-8 (neutrophil collagenase) activity was evaluated using an MMP-8 fluorimetric drug discovery kit (Enzo Life Sciences, Inc., BML-AK415-0001), and MMP-1 (fibroblast collagenase) activity was evaluated using an MMP-1 fluorimetric drug discovery kit (Enzo Life Sciences, Inc., BML-AK405-0001), in the following manner.

20 µl of each prepared evaluation material solution was individually mixed with 0.1 µl of MMP-8 included in the kit and 69.9 µl of the buffer included in the kit or with 0.2 µl of MMP-1 included in the kit and 69.8 µl of the buffer included in the kit, and a reaction was allowed to proceed at 37°C for 60 minutes. As positive controls (Cont.), samples to which no evaluation material was added and an equal amount of buffer was added were prepared. In addition, to measure the background of a substrate alone, samples (NC) in which an equal amount of buffer was added without adding MMP-8 or MMP-1 were prepared.

The fluorescent substrate included in each kit was diluted 10-fold using the buffer, and added in an amount of 10 µl to each solution after the reaction for 60 minutes. After the addition, a reaction was allowed to proceed at 37°C for 20 minutes for MMP-8 or at 37°C for 60 minutes for MMP-1, and the fluorescence intensity (excitation light: 328 nm; emission light: 420 nm) was measured. The ratio of the fluorescence intensity of each evaluation material solution-added sample minus the fluorescence intensity of the substrate alone (NC) was calculated as enzyme activity, based on the value obtained by subtracting the fluorescence intensity of the substrate alone (NC) from the fluorescence intensity of the positive control sample (Cont.) taken as 100%. Regarding the reactivity to the reaction substrate included in each kit, the reactivity of MMP-8 was higher than that of MMP-1. For this reason, the test for MMP-8 and MMP-1 was performed at different dilution rates and reaction times in order to examine reactivity under similar conditions. Figs. 4 and 5 show the results. The concentration of each evaluation material in Figs. 4 and 5 is the concentration at the time of preparing the evaluation material, and the final concentration (concentration at the time of measuring fluorescence intensity) is 1/5 of this concentration value.

As shown in Figs. 4 and 5, it was confirmed that EPC-K inhibited MMP activity (MMP-8 and MMP-1) in a concentration-dependent manner. Similar effects were not observed for ascorbic acid, or tocopherol derivatives VEA or VEN.

### 3. Cytotoxicity Evaluation Test

### Cell Culture

An oral cavity-derived epithelial cell line (Ca9-22 cells) was cultured in a 96-well plate to confluency.

### Material Preparation

Evaluation solutions were prepared using PBS containing 0.5% DMSO as a solvent so that the final concentration of each surfactant was 1%. The surfactants used were polyoxyethylene (9) lauryl ether, polyoxyethylene (20) cetyl ether, polyoxyethylene (20) stearyl ether, polyoxyethylene (10) octylphenyl ether, polyoxyethylene glycol monolaurate, sorbeth-60 tetraoleate, diethyl sebacate, and polyoxyethylene (10) hydrogenated castor oil.

### Cytotoxicity Evaluation

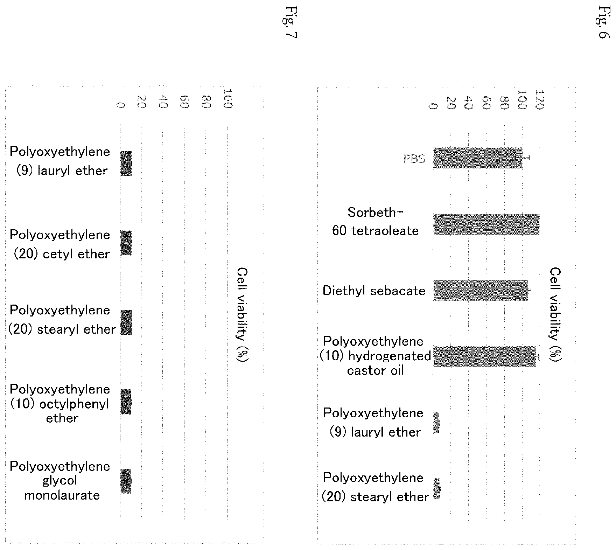

100 µl of each prepared evaluation solution was individually added to the cultured Ca9-22 cells and allowed to stand for 10 minutes at room temperature. After 10 minutes, washing was performed three times with 200 ul of PBS, a WST-1 reagent (Takara Bio Inc.) was added, and a reaction was allowed to proceed for 30 minutes. After the reaction, the absorbance at 450 nm and 600 nm was measured, and the absorbance at 600 nm was subtracted from the absorbance at 450 nm. The cell viability was calculated from the value calculated by the subtraction, based on the value obtained when only the solvent was added for the treatment taken as 100%. Figs. 6 and 7 show the results.

As shown in Figs. 6 and 7, it was confirmed that for the polyoxyethylene alkyl phenyl ether (polyoxyethylene (10) octylphenyl ether), polyoxyethylene alkyl ethers (polyoxyethylene (9) lauryl ether, polyoxyethylene (20) cetyl ether, and polyoxyethylene (20) stearyl ether), and polyoxyethylene fatty acid ester (polyoxyethylene glycol monolaurate), cytotoxicity was caused in the oral cavity-derived cells. In contrast, it was confirmed that for the other surfactants, i.e., sorbeth-60 tetraoleate, diethyl sebacate, and polyoxyethylene (10) hydrogenated castor oil, cytotoxicity was not caused in the oral cavity-derived cells.

## Claims

1. An oral composition for humans, comprising an L-ascorbic acid dl-α-tocopherol phosphoric acid diester alkali metal salt, with the proviso that an oral composition comprising at least one member selected from the group consisting of polyoxyethylene alkyl phenyl ethers, polyoxyethylene alkyl ethers, and polyoxyethylene fatty acid esters is excluded.

2. An anti-periodontal disease composition for humans, comprising an L-ascorbic acid dl-α-tocopherol phosphoric acid diester alkali metal salt.

3. The composition according to claim 1 or 2, comprising 0.001 to 1 mass% of the L-ascorbic acid dl-α-tocopherol phosphoric acid diester alkali metal salt.

4. The composition according to any one of claims 1 to 3, wherein the L-ascorbic acid dl-α-tocopherol phosphoric acid diester alkali metal salt is L-ascorbic acid dl-α-tocopherol phosphoric acid diester potassium salt.

5. The composition according to any one of claims 1 to 4, for use in anti-oral mucositis or anti-caries.

6. The composition according to any one of claims 1 to 5, for use in enhancing an oral mucosal epithelial barrier function.

7. The composition according to any one of claims 1 to 6, for use in inhibiting matrix metalloproteinase activity in the oral cavity.
